# EUROPEAN PATENT SPECIFICATION

(11) **EP 1 784 484 B1**
(45) Date of publication and mention of the grant of the patent: **18.05.2016**
(21) Application number: 05783354.3
(22) Date of filing: 02.09.2005
(51) Int. Cl.: C12N 11/08, B29B 17/00, B29C 43/00, B29C 49/00

(54) **PROCESS FOR MANUFACTURING HYDROPHOBIC POLYMERS**
VERFAHREN ZUR HERSTELLUNG VON HYDROPHOBEN POLYMEREN
PROCEDE DE PRODUCTION DE POLYMERES HYDROPHOBES

(30) Priority: 03.09.2004 BE 200400431
(43) Date of publication of application: 16.05.2007
(73) Proprietor: RESILUX, B-9230 Wetteren (BE)
(72) Inventor: DE CUYPER, Dirk, B-9070 Heusden (BE); DIERICKX, William, B-9070 Destelbergen (BE); DIERICKX, Yvan, B-9070 Heusden (BE); MERTENS, Dirk, B-9230 Wetteren ten Ede (BE); MERTENS, Johan, B-9230 Wetteren ten Ede (BE); BELADJAL, Lynda, B-9320 Wetteren ten Ede (BE)
(74) Representative: Petsis, Christos
(86) International application number: PCT/BE2005/000133
(87) International publication number: WO 2006/024115

(56) References cited:
- EP-A- 1 221 413
- EP-A- 1 375 616
- US-A- 5 618 412
- US-A1- 2004 228 828
- DATABASE WPI Section Ch, Week 200404 Derwent Publications Ltd., London, GB; Class A92, AN 2004-037846 XP002360063 & JP 2002 274521 A (YOSHINO KOGYOSHO CO LTD) 25 September 2002 (2002-09-25)

## Description

### Field of the invention

The present invention relates to a method for producing polymers incorporating living organisms and/or cell products.

### Prior art

This type of method is known for non-permanent, biodegradable hydrophilic polymers with a melting point well below 100°C, in which temperature-sensitive tissue cells and organic molecules are incorporated. The polymer degrades after a short time.

Patent US-5,985,354 in the name of MATHIOWITZ describes a method of the above type. The problem encountered in the known prior art for the incorporation of living material is that the melting point of permanent, non-biodegradable polymers is well above 100°C at normal pressure conditions. The implantation of living, active organisms or microorganisms at temperatures of this level is impossible without fatal consequences for these organisms. Accordingly, it has to be assumed that the incorporation of living material during the production of a usable object from a base material such as polymer is not achievable, even in case the organisms which are introduced can subsequently perform useful activity at normal ambient temperatures. Possible activities in this respect are oxygen consumption or absorption, absorption of radiant energy, including what is known as "UV blocking", and the like. The above therefore demonstrates that currently there are considerable restrictions in the possible range of applications for living cells in this type of polymer.

JP 2002274521 discloses a container made from biodegradable polymer including a coating layer of diamond like carbon/silicon dioxide, with formation of silicon carbide on the main body of the container aiming at preventing permeation of gas/water content to the inner surface of said container. However, said document does not disclose any solution for micro-organisms to be incorporated in the polymer. The polymer used for the container are biodegradable, so that they can be degraded by micro-organisms. In addition, the polymer can be a microbial polymer, which is a polymer made by micro-organisms. But there is no teaching as to how the man of the art could improve barrier properties by incorporating living organisms into the polymer.

EP 1 375 616 A of KIRIN BEER merely discloses working temperature ranges of 80°C, without guaranteing an activity of the micro-organisms after such a treatment, with the consequence that it lacks in providing a suitable working temperature range able to solve the problem indicated above. In addition the micro-organisms are connected to materials like glucose, mannose and others, which do not nessecitate high temperatures for their production.

As to US 5 618 412 A of HERDING et al. it discloses a fixed-bed bioreactor for purifying fluids with the aid of micro-organisms wherein carrier bodies have a porous structure with pores adapted to be penetrated by the fluid and to have micro-organisms attached thereto. This document therefore addresses a different field, which results in the proposed structure being presented with a passing action consisting in a purifying effect of passing fluid. This is in opposition to the present invention in which the aim is to block the passage so that there is a functional antagonism between the teaching of said document intended for purifying fluids passing through the proposed device in opposition to the present invention the main aim whereof is to block the passage of undesired fluids, more particularly gasses. In addition, in the solution of this document the micro-organisms are attached to the polymer particles, either between particles or inside the pores, but allways outside the material itself. Thus no teaching is disclosed as to how incorporate micro-organisms into the material of polymer.

A further problem encountered in the known prior art is that the slow diffusion of cellular components and biomolecules in a moist environment is based on a technology which is predicated on multi-wall microcapsules of hydrophilic, soluble or biodegradable polymers. However, there is currently no available technology which permits the slow diffusion of a gamma to biomolecules from a permanent polymeric carrier without it being degraded in an aqueous and/or dry environment. This applies in particular to biomolecules of the fatty acid type, such as lipids and hydrocarbons. It is therefor desirable to find a technology allowing to incorporate micro-organisms into hydrophobic or non biodegradable polymers, in order to allow such applications.

Therefore, the known prior art mainly has the following shortcoming, namely that bio-encapsulation of cells in polymers is not possible around or above 100°C under standard pressure conditions.

In US 5284761, an encapsulating method of cells in a tubular extrudate is described. In this case, both materials remain completely separated. This is completely different however from polymer-bio aggregates to be formed by complete mixing of a polymer and selected organisms; wherein cells are encapsulated by co-extrusion of a liquid cell dispersion with a polymer, wherein the polymer forms an outer coating around the liquid dispersion.

Document XP009125355 merely describes the survival of Bacillus stearothermophilus spores during a polyethylene injection process. In this research article, a link is made with the potential survival of bacterial spores during a packaging production process, and a possible contamination of food products packed in such packaging. However, no new function of the said spores to the said packaging structure is disclosed.

### Object of the invention

It is an object of the present invention to offer a solution to the abovementioned drawbacks and/or shortcomings.

### Summary of the invention

According to the present invention, there is proposed a method according to claim 1. This is made possible by the fact that the abovementioned cells and/or cells products and/or living organisms are selected from the category of what are known as the cysts and/or in a phase of inactive or dormant stages. A quite significant number of types of organisms or microorganisms can change from an active life form to what is known as a quiescent stage or spore, known as cysts. Said spores are able to withstand extreme environmental fluctuations in a latent form. In this state of anabiosis, they are able to withstand extremely dry conditions and temperatures well above 100°C.

Under suitable biotechnology conditions, these types can not only be cultivated but also converted, in controlled culture conditions, known as encysting, into usable spores for bio-encapsulation in a polymeric matrix.

During the production process of an industrial product, such as packaging material, textile fibers, granules or the like, said spores and the polymer are agglomerated within a short period of time during which the polymer is liquid, namely at a temperature above its melting point. This produces what is known as a polymer-bio aggregate, referred to below as "PBA".

As long as the product is not in use, the organisms of the bio-component in said PBA remain inactive. However, as soon as the living conditions become favorable, coinciding with the product starting to be used in association with an environment which is suitable for life in terms of temperature and relative humidity, the spores change into active, metabolizing cells under these favorable ambient conditions. For this period, the biologically active form will perform its intended function. As soon as the optimum conditions revert to conditions which are less than optimum, the active form returns to the spore.

The process remains reversible in accordance with a feedback mechanism which is controlled by the living environment of the organism in said PBA.

Thus, according to a particularly preferred embodiment of the invention, said cell products are selected from the category of so-called metabolites, i.e. the molecules which are biochemically synthesized by organisms under the abovementioned temperature working conditions.

According to yet another preferred embodiment of the present invention, the polymers are selected from non-biodegradable polymers. Reliable, slow and prolonged diffusion of organic molecules out of polymers into a moist or fluctuating environment can be realized without degradation of the polymer.

An advantage obtained by virtue of the method defined by the present invention is mainly that the biological activity of the organisms incorporated in the so-called PBA produced in accordance with the invention imparts novel, previously unknown properties to the polymer. Said PBA hereby ensures the desired environment for which said PBA was made.

A further advantage consists in the standardized release of very specific biomolecules, such as so-called repellents, from a polymeric matrix, such as granules, textile fibers and the like, without said polymeric matrix being lost in a variable environment as a result of climatological instabilities, for example. Further features and properties of the present invention are defined in further subclaims.

Further details and particularities will emerge from the following description of a number of exemplary embodiments of the method according to the invention and its uses.

### Description

In general terms, the present invention relates to a method for producing polymers which incorporate cells, living organisms and/or cell products. A number of specific use examples are described below.

A particularly significant application area is in the food packaging sector which employs what is known as an oxygen barrier, with a PBA layer arranged as an intermediate layer in multi-layer packaging material for foodstuffs, such as PET bottles for beverages, such as beers or fruit juices for example. The polymer component of the PBA is in this case PET, while the PBA bio component is a type of yeast with a dry spore, such as for example *Saccharomyces,* which is able to withstand the high temperatures of the production process. The PBA remains inactive until the PET bottle has been filled. When the package is being filled with fruit juices or beer, for example, the internal environment of the PBA becomes water-saturated, with the result that the spores are activated to form respiring cells which consume all the oxygen present inside the bottle. As a result, all the oxygen is withdrawn from the contents under the influence of what is known as the O₂ scavenger. Also, all the external oxygen which can diffuse through the wall is captured by the yeast cells for respiration, which results in an efficient oxygen barrier.

A further example of a use consists in the action as a UV blocker, which works in a similar way to the above example. In this example, instead of the yeast cells, there is incorporated in the PBA a type of alga, such as for example *Haematococcus,* the spores of which very intensively block UV light. A continuous layer of *Haematococcus* cells, haematocysts with a high concentration of astaxanthin, makes the PBA opaque to UV light. This fact is utilized in moisture-resistant UV-proof films and polymer coverings.

A still further use consists in the combined application of both examples mentioned above in connection with food packaging with an oxygen barrier and a UV blocker which is suitable for PET bottles as packaging for beers and fruit juices and the like. The PBA biocomponent is a calibrated mixture of *Saccharomyces* and *Haematococcus.* Said oxygen scavengers, such as yeast cells for example, represent a permanent oxygen barrier, while the UV blocker, such as a type of alga, for example, prevents photochemical degradation of the filling.

Yet another application consists in the absorption of energy from sunlight with a cooling effect which is similar to the example above relating to the so-called UV blocker. Instead of *Haematococcus,* the PBA incorporates a type of alga such as for example *Chlorococcus,* the active form of which, in the presence of a high degree of moisture, participates very intensively in photosynthesis, consuming high-energy rays of the sunlight. A continuous layer of cells will provide the PBA with an energy-absorbing function, resulting in a non-heating, in other words cooling, effect at the bottom of the polymer. The above effect is utilized in moisture-resistant films and polymer coverings for sun-shielding purposes.

Finally there is the application example ranging from energy-absorbing cloth to perspiration-sensitive sports clothing which derives from the previous example. A PBA with a polymer component of polypropylene and a biocomponent of photosynthesizing organisms, such as a cyanobacterium or a unicellular alga type, is extruded to form a textile fiber. The temperature-resistant spores of the algae, after they have been extruded to form a fiber, are processed to produce a textile product. Use of fibers of this type in textile products ranges from covering fabrics, such as canvas, to sports clothing. For the absorption of moisture, for example sweat, the incorporated cells will convert the incident energy of sunlight into photosynthetic metabolites. As a result, the incident solar radiation is not converted into heat, but rather is extracted from the textile fiber, resulting in the desired cooling action. When drying out when no further sweat is being produced, the cells revert to their latent, inactive state. This is because the process is reversible.

Application examples relating to slow diffusion of cellular components and at least partially biomolecules in a moist environment are described below.

In a variant on the UV blocker from the above example, the active metabolite, astaxanthin, which very intensively blocks UV light, is incorporated in the PBA instead of the *Haematococcus* cells. As an alternative to the expensive component astaxanthin, it may be possible to use less expensive UV blockers. The diffusion rate of the UV blocker from the PBA in the middle layer of the polylamellar film to the periphery is regulated at a low to very low diffusion rate, depending on the quality and requirements. This fact is exploited in moisture-resistant UV-repellent films and polymer coverings, as well as for packaging material for food products. The polymer must in this case be durable and must not deteriorate in moist conditions.

In this context, an additional example of application consists in insect-repellent films, fibers - textile - and microgranules. This represents a variant on the above example. In this case, the bio component of the PBA is a bio-active organic molecule or a mixture of molecules, preferably substances, such as lipids, fatty acids, isoprene derivatives and hydrocarbons. In addition to a film or laminate, the processed product may also be a PBA which is processed to form a textile fiber or granule or microgranule, in which the biocomponent is released to the environment at a predetermined rate. This component has a specific repellent action to insects. Examples which have been tested include:
PBA with isoprene derivates and/or branched hydrocarbons with a repellent activity to house dust mites. The PBA is extruded to form a textile fiber for weaving a fixed carpet and other products which have to be resistant to house dust mites; and
PBA with fatty acid components which are repellent to diptera, namely flies and mosquitoes, and biting and blood-sucking lice, Mallophaga, Anoplura, respectively, as well as the human head louse and poultry lice, cockroaches, ants and wasps. The PBA is granulated or extruded to form a textile fiber. The laden granules are mixed into the animal's coat, to protect against myiasis, horsefly and the like, or are scattered on the nesting site of the host of the parasite in question. Laden fibers are processed to form a protective textile as a nightcap to kill head lice, or what is known as a tissue with which an object can be rubbed to protect against ants, cockroaches, flies and the like.

Further to the above example, another important application is the use of the PBA's as a crop protection agent, in particular as a herbicide or even as a fungicide.

The biological activity of the organisms incorporated in the PBA gives the polymer new properties which were not previously known. The PBA ensures the desired environment for which the PBA was made, such as for example an anaerobic environment, complete oxygen barrier, energy absorption of solar radiation, controlled release of metabolites and the like.

The interaction and exchange of various types of organisms or microorganisms and/or molecules in the bio component of the PBA can also yield a large number of possible applications.

## Claims

1. Method for producing hydrophobic polymers incorporating living material, wherein
- a polymer is selected,
- a set of organisms is selected from among cells, living organisms and/or cell products,
- aggregates are formed by implanting said cells living organisms and/or cell products in said polymers resulting in the formation of a so-called polymer-bio aggregate, **characterized in that** implantation is carried out in the operating temperature range taken from the temperature interval of which the lower limit is set at substantially 100°C under substantially standard pressure conditions, in particular at substantially one atmosphere, wherein said cells and/or cells products, and/or living organisms are selected from among the category of the so-called cysts and/or in a phase of inactive or sleeping stages.

2. Method according to the preceding claim, **characterized in that** the cells are selected from among the prokaryotes, in particular bacteria, and/or eukaryotes.

3. Method according to the preceding claim, **characterized in that** the cells are selected from among the eukaryotes of the type protists, fungi, plants, and/or animals.

4. Method according to one of the preceding claims, **characterized in that** said cells products are selected from among the category of the so-called metabolites, being the molecules which are biochemically synthesized by organisms.

5. Method according to one of the claims 1 to 4, **characterized in that** said organisms are unicellular.

6. Method according to one of the claims 1 to 5, **characterized in that** said organisms are multicellular.

7. Method according to one of the preceding claims, **characterized in that** the polymers are selected from among non-biodegradable polymers.

8. Method according to one of the preceding claims, **characterized in that** the polymers are selected from among the family of the polyolefins.

9. Method according to the preceding claim, **characterized in that** the polymers are selected from among the family of the polyethylenes.

10. Method according to the preceding claim, **characterized in that** PET is selected from among the polymers.

11. Method according to claim 8, **characterized in that** the polymers are selected from among the family of the polypropylenes.

12. Method according to one of the claims 1 to 7, **characterized in that** the polymers are selected from among the family of the polyesters.

13. Method according to one of the preceding claims, **characterized in that** said cells and/or cell products are imbedded in said polymer.

14. Method according to one of the preceding claims, **characterized in that** said biopolymer is obtained from bringing up the cells and/or cell products while producing the polymer itself, wherein said biopolymer is obtained from a synthesis of said basis products.

15. Method according to one of the claims 1 to 13, **characterized in that** said cells and/or cell products are blended into an existing polymer, wherein said blending is performed thermally.

16. Method according to one of the claims 1 to 13, **characterized in that** said cells and/or cell products are blended in an existing polymer, wherein said blending is performed warm as intermediate layer.

17. Method according to one of the preceding claims, **characterized in that** the cysts and the polymer are agglomerated within a short period of time during which the polymer is liquid, i.e. with its temperature above its melting point.

18. Method according to one of the claims 1 to 13, **characterized in that** said cells and/or cell products are blended into an existing polymer, wherein said blending is performed cold as intermediate layer.

19. Method according to one of the preceding claims, **characterized in that** a so-called PBA layer is arranged as an intermediate layer in a multilayer packaging material for foodstuffs, in particular PET bottles for beverages.

20. Method according to the preceding claim, **characterized in that** the polymer component of the so-called PBA is composed of PET, whereas the so-called PBA biocomponent is a type of yeast with a dry spore, which is able to withstand the high temperatures of the production process.

21. Method according to the preceding claim, **characterized in that** instead of and/or possibly in combination with the yeast cells, there is incorporated a type of alga in the PBA, the spores of which block UV-light very intensively.

22. Use of the polymer - bio - aggregate obtained according to a process as defined in one of the preceding claims, as a barrier.

23. Use of the polymer - bio - aggregate obtained according to a process as defined in one of the claims 1 to 18, as an insect - repellent agent.

24. Use of the polymer - bio - aggregate obtained according to a process as defined in one of the claims 1 to 18, as a crop protection agent, in particular as an herbicide.

25. Use of the polymer - bio - aggregate obtained according to a process as defined in one of the claims 1 to 18, as a fungicide.

## Patentansprüche

1. Verfahren zur Herstellung von hydrophobe Polymeren, die lebendes Material enthalten, wobei
- ein Polymer ausgewählt wird,
- ein Satz von Organismen ausgewählt wird aus Zellen, lebenden Organismen und/oder Zellprodukten,
- Aggregate geformt werden, indem die Zellen und/oder lebenden Organismen und/oder Zellprodukten in den Polymeren implantiert werden, was in der Bildung eines sogenannten Polymer-Bio-Aggregates resultiert, **dadurch gekennzeichnet, dass** die Implantation in einem Arbeitstemperaturbereich ausgeführt wird, der aus dem Temperaturintervall ausgewählt wird, dessen unteres Limit bei im Wesentlichen 100°C liegt und bei im Wesentlichen Standarddruckbedingungen, insbesondere bei im Wesentlichen einer Atmosphäre, wobei die Zellen, und/oder Zellprodukten, und/oder lebenden Organismen aus der Kategorie der sogenannten Zysten und/oder in einer Phase der Inaktivität oder des Schlafzustandes befindlichen Zellen ausgewählt werden.

2. Verfahren nach dem voranstehenden Anspruch, **dadurch gekennzeichnet, dass** die Zellen aus Prokaryoten, insbesondere Bakterien, und/oder Eukaryoten ausgewählt werden.

3. Verfahren nach dem voranstehenden Anspruch, **dadurch gekennzeichnet, dass** die Zellen aus Eukaryoten der Art Protisten, Pilze, Pflanzen und/oder Tiere ausgewählt werden.

4. Verfahren nach einem der voranstehenden Ansprüche, **dadurch gekennzeichnet, dass** die Zellprodukte, aus der Kategorie der sogenannten Metaboliten ausgewählt werden, das sind Moleküle, die biochemisch durch Organismen synthetisiert werden.

5. Verfahren nach einem der Ansprüche 1 bis 4, **dadurch gekennzeichnet, dass** die Organismen einzellig werden.

6. Verfahren nach einem der Ansprüche 1 bis 5, **dadurch gekennzeichnet, dass** die Organismen mehrzellig werden.

7. Verfahren nach einem der voranstehenden Ansprüche, **dadurch gekennzeichnet, dass** die Polymere aus den nicht-bioabbaubaren Polymeren ausgewählt werden.

8. Verfahren nach einem der voranstehenden Ansprüche, **dadurch gekennzeichnet, dass** die Polymere aus der Familie der Polyolefine ausgewählt werden.

9. Verfahren nach dem voranstehenden Anspruch, **dadurch gekennzeichnet, dass** die Polymere aus der Familie der Polyethylene ausgewählt werden.

10. Verfahren nach dem voranstehenden Anspruch, **dadurch gekennzeichnet, dass** das PET aus den Polymeren ausgewählt wird.

11. Verfahren nach Anspruch 8, **dadurch gekennzeichnet, dass** die Polymere aus der Familie des Polypropylens ausgewählt werden.

12. Verfahren nach einem der Ansprüche 1 bis 7, **dadurch gekennzeichnet, dass** die Polymere aus der Familie der Polyester ausgewählt werden.

13. Verfahren nach einem der voranstehenden Ansprüche, **dadurch gekennzeichnet, dass** die Zellen und/oder Zellprodukte in die Polymere eingebettet werden.

14. Verfahren nach einem der voranstehenden Ansprüche, **dadurch gekennzeichnet, dass** das Biopolymer erhalten wird, indem die Zellen und/oder Zellprodukte aufgezogen werden, während das Polymer selbst hergestellt wird, wobei das Biopolymer aus einer Synthese der Basisprodukte erhalten wird.

15. Verfahren nach einem der Ansprüche 1 bis 13, **dadurch gekennzeichnet, dass** die Zellen und/oder Zellprodukte in ein bestehendes Polymer eingebracht werden, wobei das Einbringen thermisch erfolgt.

16. Verfahren nach einem der Ansprüche 1 bis 13, **dadurch gekennzeichnet, dass** die Zellen und/oder Zellprodukte in ein bestehendes Polymer eingebracht werden, wobei das Einbringen warm als Zwischenschichte erfolgt.

17. Verfahren nach einem der voranstehenden Ansprüche, **dadurch gekennzeichnet, dass** die Zysten und das Polymer in einer kurzen Zeitperiode, während der das Polymer flüssig ist, das heißt, wenn seine Temperatur oberhalb seines Schmelzpunktes liegt, agglomeriert werden.

18. Verfahren nach einem der Ansprüche 1 bis 13, **dadurch gekennzeichnet, dass** die Zellen und/oder Zellprodukte in ein bestehendes Polymer eingebracht werden, wobei das Einbringen kalt als Zwischenschichte erfolgt.

19. Verfahren nach einem der voranstehenden Ansprüche, **dadurch gekennzeichnet, dass** eine sogenannte PBA-Schicht als Zwischenlage in einem mehrlagigen Verpackungsmaterial für Lebensmittel, insbesondere PET-Flaschen für Getränke, angeordnet ist.

20. Verfahren nach dem voranstehenden Anspruch, **dadurch gekennzeichnet, dass** die Polymer-Komponente des sogenannten PBA aus PET zusammengesetzt ist, wobei die sogenannte PBA-Biokomponente eine Type von Hefe mit Trockensporen ist, die in der Lage sind, den hohen Temperaturen des Herstellungsverfahrens zu widerstehen.

21. Verfahren nach dem voranstehenden Anspruch, **dadurch gekennzeichnet, dass** an Stelle von und/oder möglicherweise in Verbindung mit den Hefezellen eine Type von Algen in das PBA eingebracht ist, deren Sporen UV-Licht sehr intensiv blockieren.

22. Verwendung des Polymer-Bio-Aggregats, erhalten nach einem Verfahren wie in einem der voranstehenden Patentansprüche definiert, als Barriere.

23. Verwendung des Polymer-Bio-Aggregats, erhalten nach einem Verfahren wie in einem der Patentansprüche 1 bis 18 definiert, als Insekten-Abweisungsmittel.

24. Verwendung des Polymer-Bio-Aggregats, erhalten nach einem Verfahren wie in einem der Ansprüche 1 bis 18 definiert, als Pflanzenschutzmittel, insbesondere als Herbizid.

25. Verwendung des Polymer-Bio-Aggregats, erhalten nach einem Verfahren wie in einem der Ansprüche 1 bis 18 defniert, als Fungizid.

## Revendications

1. Procédé de production de polymères hydrophobes incluant de la matière vivante, dans lequel
- un polymère est sélectionné,
- un ensemble d'organismes est sélectionné parmi des cellules, des organismes vivants et/ou des produits cellulaires,
- des agrégats sont formés par l'implantation desdites cellules, et/ou des organismes vivants et/ou des produits cellulaires dans lesdits polymères conduisant à la formation de ce qu'on appelle un agrégat de biopolymère, **caractérisé en ce que** l'implantation est effectuée dans la plage de température opérationnelle prise à partir de l'intervalle de température dont la limite inférieure est fixée à pratiquement 100°C sous des conditions de pression sensiblement normales, en particulier à pratiquement une atmosphère, lesdites cellules, et/ou produits cellulaires, et/ou organismes vivants étant sélectionnés parmi la catégorie de dénommés kystes et/ou dans une phase de stades inactifs ou dormants.

2. Procédé selon la revendication précédente, **caractérisé en ce que** les cellules sont sélectionnées parmi les procaryotes, en particulier des bactéries, et/ou des eucaryotes.

3. Procédé selon la revendication précédente, **caractérisé en ce que** les cellules sont sélectionnées parmi les eucaryotes du type des protistes, des champignons, des plantes et/ou des animaux.

4. Procédé selon l'une des revendications précédentes, **caractérisé en ce que** lesdits produits cellulaires sont sélectionnés parmi la catégorie des dénommés métabolites, étant les molécules qui sont synthétisées biochimiquement par des organismes.

5. Procédé selon l'une quelconque des revendications 1 à 4, **caractérisé en ce que** lesdits organismes sont unicellulaires.

6. Procédé selon l'une quelconque des revendications 1 à 4, **caractérisé en ce que** lesdits organismes sont multicellulaires.

7. Procédé selon l'une quelconque des revendications précédentes, **caractérisé en ce que** les polymères sont sélectionnés parmi des polymères non biodégradables.

8. Procédé selon l'une quelconque des revendications précédentes, **caractérisé en ce que** les polymères sont sélectionnés parmi la famille des polyoléfines.

9. Procédé selon la revendication précédente, **caractérisé en ce que** les polymères sont sélectionnés parmi la famille des polyéthylènes.

10. Procédé selon la revendication précédente, **caractérisé en ce qu'**on sélectionne le PET parmi les polymères.

11. Procédé selon la revendication 8, **caractérisé en ce que** les polymères sont sélectionnés parmi la famille des polypropylènes.

12. Procédé selon l'une quelconque des revendications 1 à 7, **caractérisé en ce que** les polymères sont sélectionnés parmi la famille des polyesters.

13. Procédé selon l'une quelconque des revendications précédentes, **caractérisé en ce que** lesdites cellules et/ou produits cellulaires sont inclus dans ledit polymère.

14. Procédé selon l'une des revendications précédentes, **caractérisé en ce que** ledit biopolymère est obtenu à partir d'une culture de cellules et/ou de produits cellulaires, tout en produisant le polymère lui-même, ledit biopolymère étant obtenu à partir d'une synthèse desdits produits de base.

15. Procédé selon l'une quelconque des revendications 1 à 13, **caractérisé en ce que** lesdites cellules et/ou produits cellulaires sont mélangés dans un polymère existant, ledit mélange étant réalisé thermiquement.

16. Procédé selon l'une quelconque des revendications 1 à 13, **caractérisé en ce que** lesdites cellules et/ou produits cellulaires sont mélangés dans un polymère existant, ledit mélange étant effectué à chaud en tant que couche intermédiaire.

17. Procédé selon l'une quelconque des revendications précédentes, **caractérisé en ce que** les kystes et le polymère sont agglomérés dans un court laps de temps pendant lequel le polymère est liquide, soit lorsque sa température est supérieure à son point de fusion.

18. Procédé selon l'une quelconque des revendications 1 à 13, **caractérisé en ce que** lesdites cellules et/ou produits cellulaires sont mélangés dans un polymère existant, ledit mélange étant effectué à froid en tant que couche intermédiaire.

19. Procédé selon l'une quelconque des revendications précédentes, **caractérisé en ce qu'**une couche d'agrégat de biopolymère dit PBA est agencée comme une couche intermédiaire dans un matériau d'emballage multicouche pour produits alimentaires, en particulier des bouteilles en PET pour boissons.

20. Procédé selon la revendication précédente, **caractérisé en ce que** le composant polymère dudit agrégat de biopolymère dit PBA est composé de PET, tandis que le biocomposant dudit PBA est un type de levure à spores sèches, qui est capable de résister aux températures élevées du processus de production.

21. Procédé selon la revendication précédente, **caractérisé en ce qu'**au lieu de et/ou éventuellement en combinaison avec les cellules de levure, il est incorporé un type d'algue dans ledit PBA, dont les spores bloquent la lumière UV très intensément.

22. Utilisation de l'agrégat de biopolymère obtenu selon un procédé tel que défini dans l'une quelconque des revendications précédentes, en tant que barrière.

23. Utilisation de l'agrégat de biopolymère obtenu selon un procédé tel que défini dans l'une quelconque des revendications 1 à 18, comme un agent insecticide.

24. Utilisation de l'agrégat de biopolymère obtenu selon un procédé tel que défini dans l'une quelconque des revendications 1 à 18, en tant qu'agent de protection des cultures, en particulier comme herbicide.

25. Utilisation de l'agrégat de biopolymère obtenu selon un procédé tel que défini dans l'une quelconque des revendications 1 à 18, en tant que fongicide.
